# EUROPEAN PATENT SPECIFICATION

(11) **EP 3 524 309 B1**
(45) Date of publication and mention of the grant of the patent: **10.04.2024**
(21) Application number: 16856462.3
(22) Date of filing: 04.10.2016
(51) Int. Cl.: A61M 25/10, A61M 25/00, A61M 25/01

(54) **CATHETER AND BALLOON CATHETER**
KATHETER UND BALLONKATHETER
CATHÉTER ET CATHÉTER À BALLONNET

(43) Date of publication of application: 14.08.2019
(73) Proprietor: Asahi Intecc Co., Ltd., Seto-shi, Aichi 489-0071 (JP)
(72) Inventor: SHIMIZU Yuusuke, Seto-shi, Aichi 489-0071 (JP); ITO Hiroshi, Seto-shi, Aichi 489-0071 (JP); HASE Shogo, Seto-shi, Aichi 489-0071 (JP)
(74) Representative: Winter, Brandl - Partnerschaft mbB
(86) International application number: PCT/JP2016/079471
(87) International publication number: WO 2018/066060

(56) References cited:
- EP-A1- 2 923 723
- WO-A1-2012/116337
- WO-A1-2015/099935
- WO-A1-2015/099935
- JP-A- 2013 005 823
- JP-A- 2014 236 863
- JP-A- 2015 147 080
- JP-A- 2015 147 080
- US-A- 4 147 169

## Description

### Field

The present invention relates to a catheter and a balloon catheter used for diagnosis or treatment of a stenosis or stricture part or an obstructed part in a blood vessel or in a digestive organ.

### Background

A stenosis or stricture part or an obstructed part that is formed within a blood vessel, a bile duct, a pancreatic duct, or the like interrupts the flow of blood, bile (biliary fluid), pancreatic juice, or the like. There is a common method of diagnosis or treatment of the stenosis or stricture part or the obstructed part, which uses a catheter.

A typical catheter comprises a tubular inner layer, an outer layer covering the outer circumference of the inner layer, and a reinforcing layer arranged between the inner layer and the outer layer. In a catheter of this type comprising a reinforcing layer interposed between an inner layer and an outer layer to bond these layers, it is difficult to strengthen the bond between the inner layer and the outer layer.

There are techniques known to solve this problem. One of the techniques is a catheter with a strengthened bond between its inner layer and its outer layer, comprising a reinforcing layer having a wavy contour, the inner layer having an outer surface that follows the wavy contour of the reinforcing layer, and the outer layer having an inner surface that follows the wavy contour of the reinforcing layer (see Patent Literature 1, for example). Another one of the techniques is a catheter with a strengthened bond between its inner layer and its outer layer, comprising a protruding portion that is disposed on the inner layer and that protrudes through a gap in a reinforcing layer toward the outer layer and extends in the axial direction to stick into the outer layer (see Patent Literature 2, for example).

However, in the catheter described in Patent Literature 1, only the irregularities are provided on the outer circumference of the inner layer and on the inner circumference of the outer layer. In the catheter described in Patent Literature 2, only the protruding portion disposed on the inner layer extends in one direction from the proximal end toward the front. Therefore, there has been a problem that the outer layer readily comes off the inner layer when the outer layer is pulled in the distal end direction due to the presence of a stenosis or stricture part or an obstructed part. This problem of the outer layer readily coming off the inner layer upon the outer layer being pulled in the distal end direction due to the presence of a stenosis or stricture part or an obstructed part is more likely to occur especially when the catheter is inserted into a curving duct such as a blood vessel, a bile duct, or a pancreatic duct and thereby the catheter bends to cause concentration of stress at the portion where the outer layer and the inner layer are bonded to each other Further prior art catheters and balloon catheters are disclosed in WO2012/116337, JP 2015 147080 and in US4147169.

### Citation List

### Patent Literature

Patent Literature 1: Japanese Patent No. 4741151
Patent Literature 2: International Publication No. WO 2015/012185

### Summary

### Technical Problem

The present invention is devised based on the above circumstances, and an object of the present invention is to provide a catheter and a balloon catheter in which an outer layer does not readily come off an inner layer when the outer layer is pulled in the axial direction (in the distal end direction and in the proximal end direction) or in the radial direction (outwardly). The invention is defined in claims 1,3.

### Solution to Problem

The above problem is solved by means listed below.

Provided in embodiment 1 of the present invention is a catheter, comprising:
a tubular inner layer;
a reinforcing layer disposed within the inner layer or on an outer circumference of the inner layer, the reinforcing layer being made of wound wires, each adjacent pair of the wires having a gap therebetween; and
a first outer layer covering the reinforcing layer, wherein:
   in the gap of the reinforcing layer, disposed is a first bonding portion where a first inner layer protruding portion of the inner layer sticking into the first outer layer is engaged with a first outer layer protruding portion of the first outer layer sticking into the inner layer.

Provided in embodiment 2 of the present invention is the catheter according to embodiment 1, further comprising:
a second outer layer covering an outer circumference of the reinforcing layer, the second outer layer being arranged on a distal end side of the first outer layer, the second outer layer being made of a resin that is more flexible than a resin of which the first outer layer is made,
wherein:
in the gap of the reinforcing layer, disposed is a second bonding portion where a second inner layer protruding portion of the inner layer sticking into the second outer layer is engaged with a second outer layer protruding portion of the second outer layer sticking into the inner layer,
a degree of the engagement of the second bonding portion is higher than a degree of the engagement of the first bonding portion.

Provided in embodiment 3 of the present invention is a balloon catheter, comprising:
a tubular inner layer;
a reinforcing layer disposed within the inner layer or on an outer circumference of the inner layer, the reinforcing layer being made of wound wires, each adjacent pair of the wires having a gap therebetween; and
a balloon covering the reinforcing layer,
wherein:
in the gap of the reinforcing layer, disposed is a bonding portion where an inner layer protruding portion of the inner layer sticking into the balloon is engaged with a balloon protruding portion of the balloon sticking into the inner layer.

### Advantageous Effects of Invention

In a catheter according to embodiment 1 of the present invention, in a gap of a reinforcing layer, disposed is a first bonding portion where a first inner layer protruding portion of an inner layer sticking into a first outer layer is engaged with a first outer layer protruding portion of the first outer layer sticking into the inner layer. This configuration enlarges the area across which the first outer layer and the inner layer are bonded to each other within the gap of the reinforcing layer, leading to a strengthened bond between the first outer layer and the inner layer. And the first bonding portion is anchored in the reinforcing layer to produce an anchoring effect, which can reduce the probability of the first outer layer to come off the inner layer when the catheter bends to cause the first outer layer to be pulled in the axial direction (in the distal end direction and in the proximal end direction). In addition, the engagement between the first inner layer protruding portion and the first outer layer protruding portion of the first bonding portion can reduce the probability of the first outer layer to come off the inner layer when the first outer layer is pulled in the radial direction (outwardly).

According to embodiment 2 of the present invention, the catheter further comprises a second outer layer covering the outer circumference of the reinforcing layer, the second outer layer being arranged on the distal end side of the first outer layer, the second outer layer being made of a resin that is more flexible than a resin of which the first outer layer is made, wherein in the gap of the reinforcing layer, disposed is a second bonding portion where a second inner layer protruding portion of the inner layer sticking into the second outer layer is engaged with a second outer layer protruding portion of the second outer layer sticking into the inner layer. A degree of the engagement of the second bonding portion is higher than a degree of the engagement of the first bonding portion (more specifically, the engaging force between the second inner layer protruding portion and the second outer layer protruding portion of the second bonding portion is stronger than the engaging force between the first inner layer protruding portion and the first outer layer protruding portion of the first bonding portion). When the catheter is inserted into a peripheral section of a blood vessel, a bile duct, a pancreatic duct, or the like, the distal end side of the catheter bends to a great extent following the curve of the blood vessel, a bile duct, a pancreatic duct, or the like to readily cause stress to be concentrated at the bonding portion where the second outer layer made of a flexible resin is bonded to the inner layer. However, the high degree of engagement between the second inner layer protruding portion and the second outer layer protruding portion of the second bonding portion (more specifically, the strong engaging force between the second inner layer protruding portion and the second outer layer protruding portion) as well as the enlarged area across which the second outer layer and the inner layer are bonded to each other in the gap of the reinforcing layer strengthen the bond between the second outer layer and the inner layer, which can lead to a reduced probability of the second outer layer made of a flexible resin to come off the inner layer.

In a balloon catheter according to embodiment 3 of the present invention, in a gap of a reinforcing layer, disposed is a bonding portion where an inner layer protruding portion of an inner layer sticking into a balloon is engaged with a balloon protruding portion of the balloon sticking into the inner layer. This configuration enlarges the area across which the balloon and the inner layer are bonded to each other within the gap of the reinforcing layer, leading to a strengthened bond between the balloon and the inner layer. And the balloon is anchored in the reinforcing layer to produce an anchoring effect, which can reduce the probability of the balloon to come off the inner layer when the balloon catheter bends to cause the balloon to be pulled in the axial direction (in the distal end direction and in the proximal end direction). In addition, the engagement between the inner layer protruding portion and the balloon protruding portion in the bonding portion can reduce the probability of the balloon to come off the inner layer when the balloon is expanded radially (outwardly). The engagement between the inner layer protruding portion and the balloon protruding portion can also allow a reduction in the thickness of the balloon while maintaining the strength of the bond between the balloon and the inner layer, leading to an enhanced insertion of the balloon catheter into a blood vessel, a bile duct, a pancreatic duct, or the like.

### Brief Description of Drawings

FIG. 1 is an overall view of a catheter according to a first embodiment.
FIG. 2 is an expanded view of the part A in FIG. 1.
FIG. 3 is a sectional view taken from B-B line in FIG. 2, showing only one of two sides (the upper side).
FIG. 4 is a sectional view of a catheter according to a second embodiment, viewed in the same manner as in FIG. 3.
FIG. 5 is a sectional view of a catheter according to a third embodiment, viewed in the same manner as in FIG. 3.
FIG. 6 is an expanded view of a catheter according to a fourth embodiment, viewed in the same manner as in FIG. 2.
FIG. 7 is a sectional view taken from C-C line in FIG. 6, showing only one of two sides (the upper side).
FIG. 8 is a sectional view of a catheter according to a fifth embodiment, viewed in the same manner as in FIG. 3.
FIG. 9 is an overall view of a balloon catheter according to a sixth embodiment.
FIG. 10 is an expanded view of the part F in FIG. 9, showing only one of two sides (the upper side).
FIG. 11 is a sectional view of a catheter according to a seventh embodiment, viewed in the same manner as in FIG. 3.
FIG. 12 is a sectional view of a catheter according to an eighth embodiment, viewed in the same manner as in FIG. 3.

### Description of Embodiments

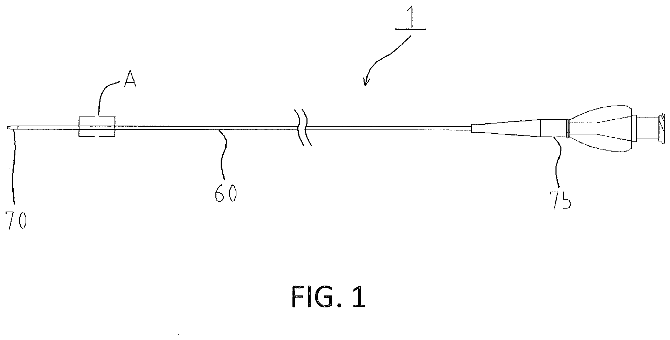

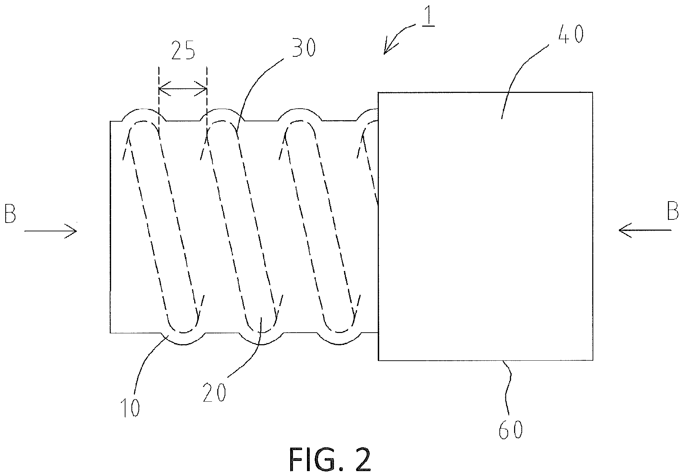

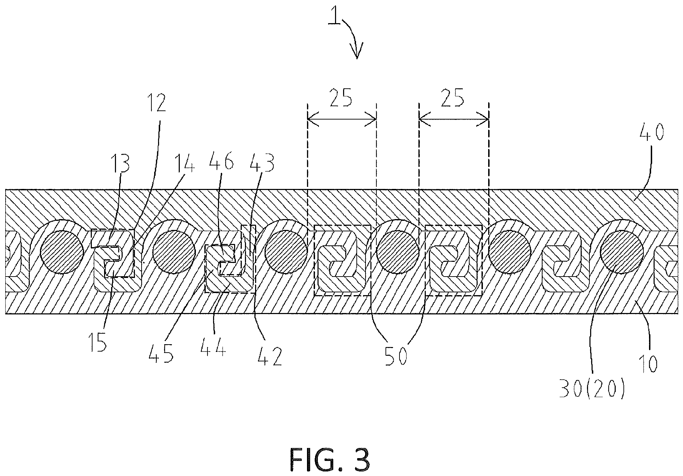

Referring to FIG. 1 to FIG. 3, a catheter 1 according to a first embodiment is described below. In FIG. 1, the distal end side (the front-end side) to be inserted into the body is shown to the left hand side, and the proximal end side (the rear-end side) to be manipulated by a handler such as a doctor is shown to the right hand side. FIG. 2 is an expanded view of the part A in FIG. 1. FIG. 3 is a sectional view taken from B-B line in FIG. 2.

The catheter 1 is, for example, a catheter used for diagnosis or treatment of a stenosis or stricture part or an obstructed part. As shown in FIG. 1, the catheter 1 essentially comprises a catheter shaft 60, a tip 70 bonded to the distal end of the catheter shaft 60, and a connector 75 bonded to the proximal end of the catheter shaft 60.

As shown in FIG. 2, the catheter shaft 60 comprises, in order from inside in the radial direction, a tubular inner layer 10, a reinforcing layer (coil body) 30 that is disposed within the inner layer 10 and made of wound wires 20 with each adjacent pair of the wires 20 having a gap 25 therebetween, and a first outer layer 40 covering the reinforcing layer (coil) 30. For easy understanding, FIG. 2 shows the catheter with the first outer layer 40 partially removed.

The inner layer 10 is made of a resin and can accommodate a guidewire or another catheter inserted thereinto. The resin material constituting the inner layer 10 is not particularly limited but PTFE (polytetrafluoroethylene) is used in the first embodiment.

The coil body 30 as the reinforcing layer is disposed in the tubular inner layer 10. The coil body 30 is made of the wires 20 that are wound in the rightward direction toward the distal end side. As the material of the wires 20 constituting the coil body 30, stainless steel (SUS304) is used in the first embodiment, but it is not limitative. The material may not only be a metal material such as tungsten or Ni-Ti alloy but also a resin material such as reinforced plastic (PEEK), for example. Alternatively, the wires 20 constituting the coil body 30 may be wound in the leftward direction toward the distal end side.

The first outer layer 40 made of a resin is disposed on the outer circumference of the reinforcing layer (coil body) 30 and covers the inner layer 10 and the reinforcing layer (coil body) 30. The resin material constituting the first outer layer 40 is not particularly limited and a polyamide, a polyamide elastomer, a polyester, or a polyurethane, for example may be used.

Bonded to the distal end of the catheter shaft 60 described above is the tip 70 made of a resin (see FIG. 1). The resin constituting the tip 70 is not particularly limited but is made of a polyurethane or a polyurethane elastomer, for example. The tip 70 may also contain a radiopaque powder. When the tip 70 contains a radiopaque powder (such as a tungsten powder) in a content from about 65 w% to about 90 w%, for example, a handler such as a doctor can keep track of the exact location of the catheter 1 during coronary angiography.

As shown in FIG. 3, disposed in the gap 25 of the reinforcing layer (coil body) 30 is a first bonding portion 50 where a first inner layer protruding portion 12 of the inner layer 10 sticking into the first outer layer 40 is engaged with a first outer layer protruding portion 42 of the first outer layer 40 sticking into the inner layer 10.

The first inner layer protruding portion 12 is composed of a proximal end direction extending portion 13 extending in the proximal end direction, an inwardly-extending portion 14 extending inwardly, and a distal end direction extending portion 15 extending in the distal end direction. The first outer layer protruding portion 42 is composed of an inwardly-protruding portion 43 extending inwardly, a distal end direction extending portion 44 extending in the distal end direction, an outwardly-extending portion 45 extending outwardly, and a proximal end direction extending portion 46 extending in the proximal end direction.

In the catheter 1, in the gap 25 of the reinforcing layer (coil body) 30, disposed is the first bonding portion 50 where the first inner layer protruding portion 12 of the inner layer 10 sticking into the first outer layer 40 is engaged with the first outer layer protruding portion 42 of the first outer layer 40 sticking into the inner layer 10. This configuration enlarges the area across which the first outer layer 40 and the inner layer 10 are bonded to each other within the gap 25 of the reinforcing layer (coil body) 30, leading to a strengthened bond between the first outer layer 40 and the inner layer 10. The first bonding portion 50 is anchored in the reinforcing layer (coil body) 30 to produce an anchoring effect, which can reduce the probability of the first outer layer 40 to come off the inner layer 10 when the catheter 1 bends to cause the first outer layer 40 to be pulled in the axial direction (in the distal end direction and in the proximal end direction). In addition, the engagement between the first inner layer protruding portion 12 and the first outer layer protruding portion 42 can reduce the probability of the first outer layer 40 to come off the inner layer 10 when the first outer layer 40 is pulled in the radial direction (outwardly).

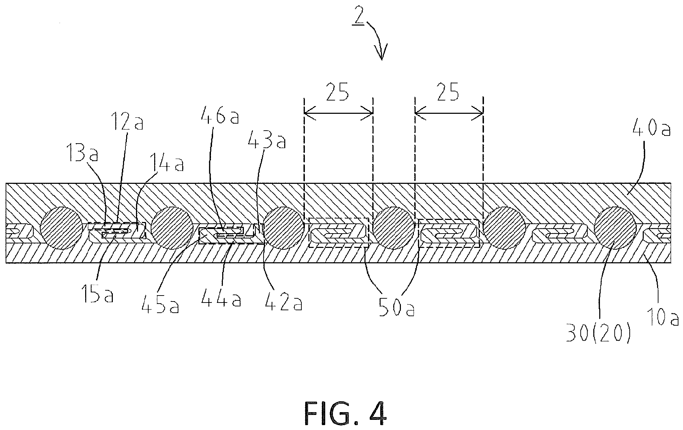

Referring to FIG. 4, a catheter 2 according to a second embodiment is described below. Description below only includes the differences from the catheter 1 shown in FIG. 3. In the catheter 2, an inward surface of the coil body 30 as the reinforcing layer is buried in an inner layer 10a and an outward surface of the coil body 30 as the reinforcing layer is buried in a first outer layer 40a. In the gap 25 of the reinforcing layer (coil body) 30, disposed is a first bonding portion 50a where a first inner layer protruding portion 12a of the inner layer 10a sticking into the first outer layer 40a is engaged with a first outer layer protruding portion 42a of the first outer layer 40a sticking into the inner layer 10a.

The first inner layer protruding portion 12a is composed of a proximal end direction extending portion 13a extending in the proximal end direction, an inwardly-extending portion 14a extending inwardly, and a distal end direction extending portion 15a extending in the distal end direction. The first outer layer protruding portion 42a is composed of an inwardly-protruding portion 43a extending toward the inner layer 10a, a distal end direction extending portion 44a extending in the distal end direction, an outwardly-extending portion 45a extending outwardly, and a proximal end direction extending portion 46a extending in the proximal end direction.

In the catheter 2, just like in the catheter 1, in the gap 25 of the reinforcing layer (coil body) 30, disposed is the first bonding portion 50a where the first inner layer protruding portion 12a of the inner layer 10a sticking into the first outer layer 40a is engaged with the first outer layer protruding portion 42a of the first outer layer 40a sticking into the inner layer 10a. This configuration enlarges the area across which the first outer layer 40a and the inner layer 10a are bonded to each other within the gap 25 of the reinforcing layer (coil body) 30, leading to a strengthened bond between the first outer layer 40a and the inner layer 10a. The first bonding portion 50a is anchored in the reinforcing layer (coil body) 30 to produce an anchoring effect, which can reduce the probability of the first outer layer 40a to come off the inner layer 10a when the catheter 2 bends to cause the first outer layer 40a to be pulled in the axial direction (in the distal end direction and in the proximal end direction). In addition, the engagement between the first inner layer protruding portion 12a and the first outer layer protruding portion 42a can reduce the probability of the first outer layer 40a to come off the inner layer 10a when the first outer layer 40a is pulled in the radial direction (outwardly).

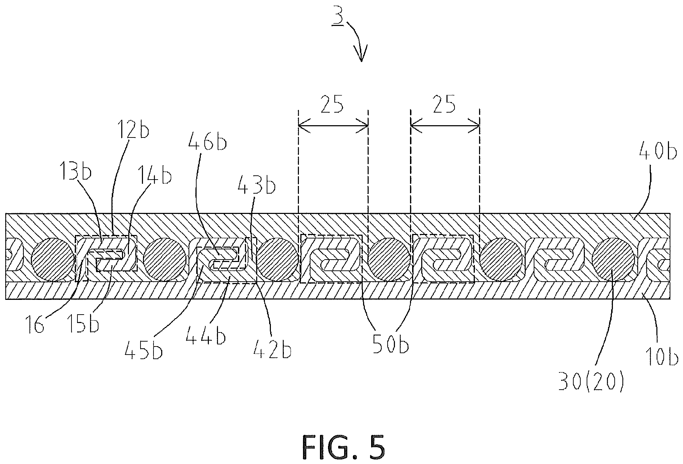

Next, referring to FIG. 5, a catheter 3 according to a third embodiment is described below. Description below only includes the differences from the catheter 1 shown in FIG. 3. In the catheter 3, the coil body 30 as the reinforcing layer is disposed on the outer circumference of an inner layer 10b. In the gap 25 of the reinforcing layer (coil body) 30, disposed is a first bonding portion 50b where a first inner layer protruding portion 12b of the inner layer 10b sticking into a first outer layer 40b is engaged with a first outer layer protruding portion 42b of the first outer layer 40b sticking into the inner layer 10b.

The first inner layer protruding portion 12b is composed of an outwardly-extending portion 16 extending outwardly, a proximal end direction extending portion 13b extending in the proximal end direction, an inwardly-extending portion 14b extending inwardly, and a distal end direction extending portion 15b extending in the distal end direction. The first outer layer protruding portion 42b is composed of an inwardly-protruding portion 43b extending inwardly, a distal end direction extending portion 44b extending in the distal end direction, an outwardly-extending portion 45b extending outwardly, and a proximal end direction extending portion 46b extending in the proximal end direction.

In the catheter 3, just like in the catheter 1, in the gap 25 of the reinforcing layer (coil body) 30, disposed is the first bonding portion 50b where the first inner layer protruding portion 12b of the inner layer 10b sticking into the first outer layer 40b is engaged with the first outer layer protruding portion 42b of the first outer layer 40b sticking into the inner layer 10b. This configuration enlarges the area across which the first outer layer 40b and the inner layer 10b are bonded to each other within the gap 25 of the reinforcing layer (coil body) 30, leading to a strengthened bond between the first outer layer 40b and the inner layer 10b. The first bonding portion 50b is anchored in the reinforcing layer (coil body) 30 to produce an anchoring effect, which can reduce the probability of the first outer layer 40b to come off the inner layer 10b when the catheter 3 bends to cause the first outer layer 40b to be pulled in the axial direction (in the distal end direction and in the proximal end direction). In addition, the engagement between the first inner layer protruding portion 12b and the first outer layer protruding portion 42b can reduce the probability of the first outer layer 40b to come off the inner layer 10b when the first outer layer 40b is pulled in the radial direction (outwardly).

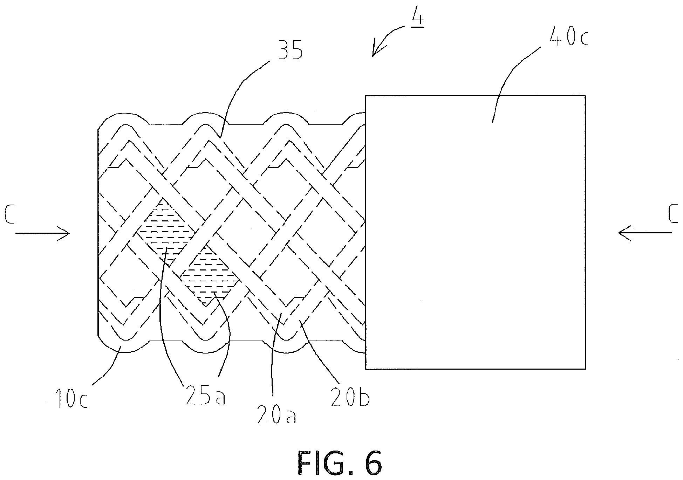

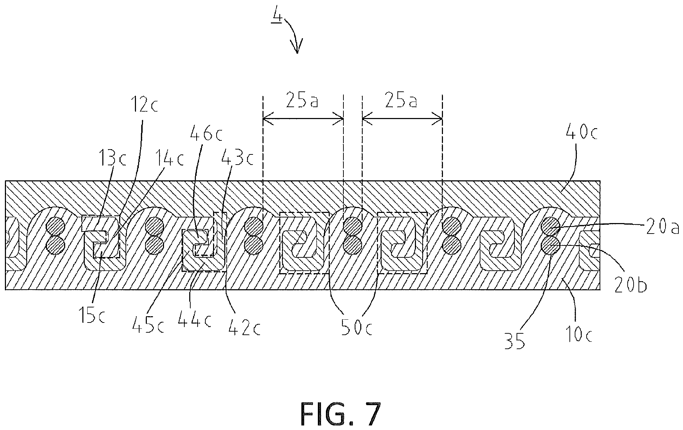

Next, referring to FIG. 6 and FIG. 7, a catheter 4 according to a fourth embodiment is described below. Description below only includes the differences from the catheter 1 shown in FIG. 2. The catheter 4 comprises, in order from inside in the radial direction, an inner layer 10c, a reinforcing layer (braid) 35 that is disposed in the inner layer 10c and is braided with a plurality of first wires 20a and second wires 20b with each adjacent pair of the first wires 20a and of the second wires 20b having a gap 25a therebetween, and an outer layer 40c covering the reinforcing layer (braid) 35 (see FIG. 6). For easy understanding, FIG. 6 shows the catheter with the outer layer 40c partially removed.

The reinforcing layer (braid) 35 is a net (mesh) braided with the first wires 20a and the second wire 20b. The first wires 20a are wound in the rightward direction toward the distal end side, and the second wires 20b are wound in the leftward direction toward the distal end side. In the fourth embodiment, eight first wires 20a and eight second wires 20b, namely, 16 (= 8 × 8) wires in total, are braided over and under each other to form the reinforcing layer (braid) 35.

The first wires 20a and the second wires 20b constituting the reinforcing layer (braid) 35 may be made of the same material or different materials. The first wires made of tungsten and the second wires made of stainless steel (SUS304) are used in the fourth embodiment, which is not limitative. Instead of a metal material, a resin material (reinforced plastic, for example) may be used.

As shown in FIG. 7, in the gap 25a of the reinforcing layer (braid) 35, disposed is a first bonding portion 50c where a first inner layer protruding portion 12c of the inner layer 10c sticking into a first outer layer 40c is engaged with a first outer layer protruding portion 42c of the first outer layer 40c sticking into the inner layer 10c.

The first inner layer protruding portion 12c is composed of a proximal end direction extending portion 13c extending in the proximal end direction, an inwardly-extending portion 14c extending inwardly, and a distal end direction extending portion 15c extending in the distal end direction. The first outer layer protruding portion 42c is composed of an inwardly-protruding portion 43c extending toward the inner layer 10c, a distal end direction extending portion 44c extending in the distal end direction, an outwardly-extending portion 45c extending outwardly, and a proximal end direction extending portion 46c extending in the proximal end direction.

In the catheter 4, in the gap 25a of the reinforcing layer (braid) 35, disposed is the first bonding portion 50c where the first inner layer protruding portion 12c of the inner layer 10c sticking into the first outer layer 40c is engaged with the first outer layer protruding portion 42c of the first outer layer 40c sticking into the inner layer 10c. This configuration enlarges the area across which the first outer layer 40c and the inner layer 10c are bonded to each other within the gap 25a of the reinforcing layer (braid) 35, leading to a strengthened bond between the first outer layer 40c and the inner layer 10c. The first bonding portion 50c is anchored in the reinforcing layer (braid) 35 to produce an anchoring effect, which can reduce the probability of the first outer layer 40c to come off the inner layer 10c when the catheter 4 bends to cause the first outer layer 40c to be pulled in the axial direction (in the distal end direction and in the proximal end direction). In addition, the engagement between the first inner layer protruding portion 12c and the first outer layer protruding portion 42c can reduce the probability of the first outer layer 40c to come off the inner layer 10c when the first outer layer 40c is pulled in the radial direction (outwardly).

The configuration of the reinforcing layer (braid) 35 of the catheter 4 may be modified in the following ways: (i) as in the catheter 2 shown in FIG. 4, an inward surface of the braid 35 as the reinforcing layer is buried in the inner layer 10c and an outward surface of the braid 35 as the reinforcing layer is buried in the first outer layer 40c; or (ii) as in the catheter 3 shown in FIG. 5, the braid 35 as the reinforcing layer is disposed on the outer circumference of the inner layer 10c.

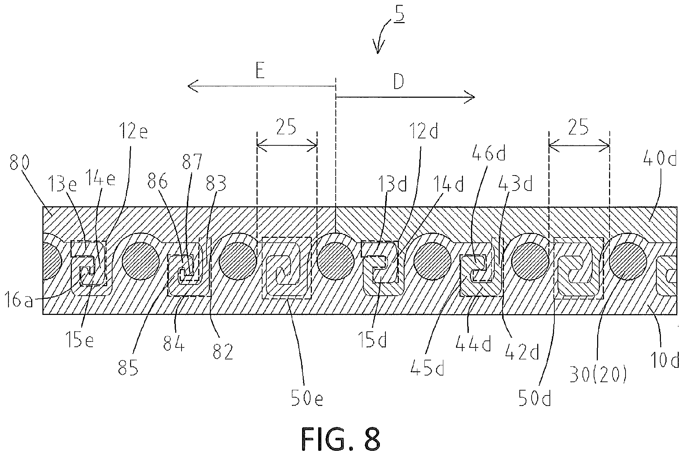

Next, referring to FIG. 8, a catheter 5 according to a fifth embodiment is described below. Description below only includes the differences from the catheter 1 shown in FIG. 3. A proximal end side D of the catheter 5 comprises, in order from inside in the radial direction, a tubular inner layer 10d, the reinforcing layer (coil body) 30 that is disposed within the inner layer 10d and made of the wound wires 20 with each adjacent pair of the wires 20 having the gap 25 therebetween, and a first outer layer 40d covering the reinforcing layer (coil body) 30. A distal end side E of this catheter comprises, in order from inside in the radial direction, the tubular inner layer 10d, the reinforcing layer (coil body) 30 that is disposed within the inner layer 10d and made of the wound wires 20 with each adjacent pair of the wires 20 having the gap 25 therebetween, and a second outer layer 80 covering the reinforcing layer (coil body) 30.

The second outer layer 80 is made of a resin more flexible than the resin constituting the first outer layer 40d. The resin material constituting the second outer layer 80 is not particularly limited and may be a polyamide, a polyamide elastomer, a polyester, or a polyurethane, for example, as in the case of the first outer layer 40d.

The proximal end side D of the catheter 5 comprises, in the gap 25 of the reinforcing layer (coil body) 30, a first bonding portion 50d where a first inner layer protruding portion 12d of the inner layer 10d sticking into the first outer layer 40d is engaged with a first outer layer protruding portion 42d of the first outer layer 40d sticking into the inner layer 10d. The distal end side E of the catheter 5 comprises, in the gap 25 of the reinforcing layer (coil body) 30, a second bonding portion 50e where a second inner layer protruding portion 12e of the inner layer 10d sticking into the second outer layer 80 is engaged with a second outer layer protruding portion 82 of the second outer layer 80 sticking into the inner layer 10d.

The first inner layer protruding portion 12d is composed of a proximal end direction extending portion 13d extending in the proximal end direction, an inwardly-extending portion 14d extending inwardly, and a distal end direction extending portion 15d extending in the distal end direction. The first outer layer protruding portion 42d is composed of an inwardly-protruding portion 43d extending inwardly, a distal end direction extending portion 44d extending in the distal end direction, an outwardly-extending portion 45d extending outwardly, and a proximal end direction extending portion 46d extending in the proximal end direction.

The second inner layer protruding portion 12e is composed of a proximal end direction extending portion 13e extending in the proximal end direction, an inwardly-extending portion 14e extending inwardly, a distal end direction extending portion 15e extending in the distal end direction, and an outwardly-extending portion 16a extending outwardly. The second outer layer protruding portion 82 is composed of an inwardly-protruding portion 83 extending inwardly, a distal end direction extending portion 84 extending in the distal end direction, an outwardly-extending portion 85 extending outwardly, a proximal end direction extending portion 86 extending in the proximal end direction, and an inwardly-protruding portion 87 extending inwardly.

In the catheter 5, the degree of the engagement of the second bonding portion 50e is higher than the degree of the engagement of the first bonding portion 50d (more specifically, the engaging force between the second inner layer protruding portion 12e and the second outer layer protruding portion 82 of the second bonding portion 50e is stronger than the engaging force between the first inner layer protruding portion 12d and the first outer layer protruding portion 42d of the first bonding portion 50d). When the catheter 5 is inserted into a peripheral section of a blood vessel, a bile duct, a pancreatic duct, or the like, the distal end side E of the catheter 5 bends to a great extent following the curve of the blood vessel, a bile duct, a pancreatic duct, or the like to readily cause stress to be concentrated at the second bonding portion 50e where the second outer layer 80 made of a flexible resin is bonded to the inner layer 10d. However, the high degree of engagement between the second inner layer protruding portion 12e and the second outer layer protruding portion 82 of the second bonding portion 50e (more specifically, the strong engaging force between the second inner layer protruding portion 12e and the second outer layer protruding portion 82) as well as the enlarged area across which the second outer layer 80 and the inner layer 10d are bonded to each other in the gap 25 of the reinforcing layer (coil body) 30 strengthen the bond between the second outer layer 80 and the inner layer 10d, which can lead to a reduced probability of the second outer layer 80 made of a flexible resin to come off the inner layer 10d.

The configuration of the reinforcing layer (coil body) 30 of the catheter 5 may be modified in the following ways: (i) as in the catheter 2 shown in FIG. 4, an inward surface of the coil body 30 as the reinforcing layer is buried in the inner layer 10d and an outward surface of the coil body 30 as the reinforcing layer is buried in the first outer layer 40d and the second outer layer 80; or (ii) as in the catheter 3 shown in FIG. 5, the coil body 30 as the reinforcing layer is disposed on the outer circumference of the inner layer 10d. In the catheter 5, the reinforcing layer (coil body) 30 may be replaced by the reinforcing layer (braid) 35 as in the catheter 4 shown in FIG. 7.

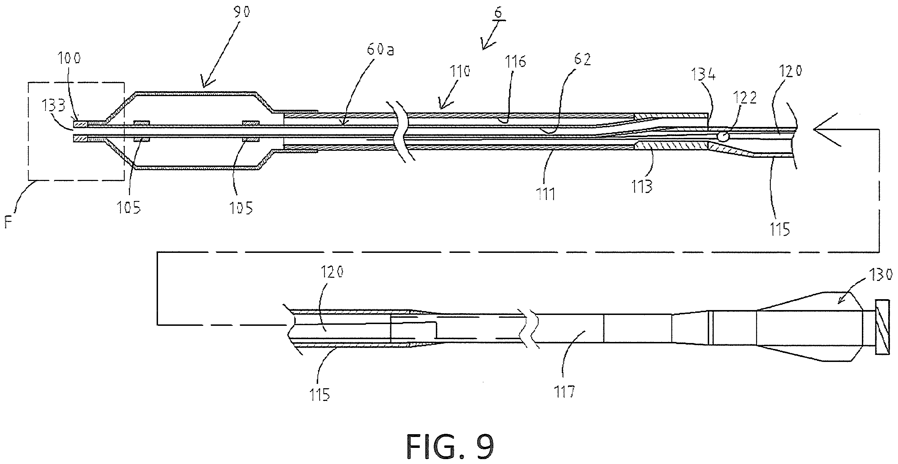

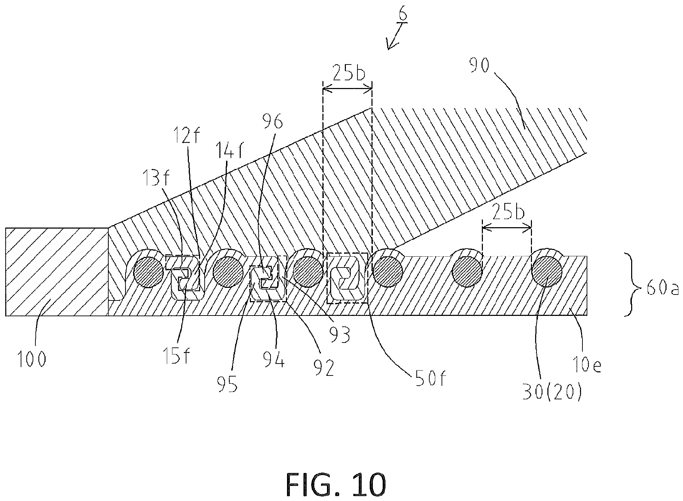

Next, referring to FIG. 9 and FIG. 10, a balloon catheter 6 according to a sixth embodiment is described below. FIG. 10 is an expanded view of the part F in FIG. 9. The balloon catheter 6 is, for example, a therapeutic balloon catheter used for dilating a stenosis or stricture part or an obstructed part for curative purposes.

As shown in FIG. 9, the balloon catheter 6 essentially comprises a balloon 90, a tip 100, an outer shaft 110, an inner shaft 60a, a reinforcing member 120, and a connector 130.

The balloon 90 for dilating a stenosis or stricture part or an obstructed part is made of a resin component. The distal end of the balloon 90 is bonded to the distal end of the inner shaft 60a and the tip 100. The proximal end of the balloon 90 is bonded to the distal end of the outer shaft 110.

The outer shaft 110 is a tubular component constituting an expandable lumen 116, which feeds liquid such as a contrast medium and physiological saline for expanding the balloon 90. The outer shaft 110 is composed of, from the distal end side, an outer-shaft distal end 111, a guidewire port 113, an outer-shaft middle portion 115, and an outer-shaft proximal end 117. Each of the outer-shaft distal end 111 and the outer-shaft middle portion 115 is a tube made of a resin such as a polyamide, a polyamide elastomer, a polyolefin, a polyester, or a polyester elastomer. The guidewire port 113 is where the outer-shaft distal end 111, the outer-shaft middle portion 115, and the inner shaft 60a are bonded to each other.

The outer-shaft distal end 111 accommodates the inner shaft 60a inserted thereinto. Between the outer-shaft distal end 111 and the inner shaft 60a, the expandable lumen 116 described above is formed.

The outer-shaft proximal end 117 is a tubular metal component, called a hypotube. The distal end of the outer-shaft proximal end 117 is inserted into and bonded to the proximal end of the outer-shaft middle portion 115. The proximal end of the outer-shaft proximal end 117 has the connector 130 attached thereto. From an indeflator (not shown) attachable to the connector 130, liquid for expanding the balloon 90 such as a contrast medium and physiological saline is fed, which travels through the expandable lumen 116 to reach the balloon 90 to expand it. The material constituting the outer-shaft proximal end 117 is not particularly limited and may be a super-elastic alloy such as stainless steel (SUS302, SUS304) or Ni-Ti alloy.

Accommodated in the interior of the inner shaft 60a is a guidewire lumen 62 into which a guidewire is inserted. The proximal end of the inner shaft 60a is bonded to the guidewire port 113 of the outer shaft 110 to form a proximal end-side guidewire port 134. Through the proximal end-side guidewire port 134, a handler can replace a guidewire.

To the distal end of the inner shaft 60a and the distal end of the balloon 90, the tip 100 is bonded. The tip 100 is made of a flexible resin. The material thereof is not particularly limited and may be a polyurethane or a polyurethane elastomer, for example. The distal end tip 100 has a distal end-side guidewire port 133 on the distal end thereof.

The reinforcing member 120 is bonded to the inner circumference of the distal end of the outer-shaft proximal end 117. The reinforcing member 120 is a tapered metal wire that has a circular cross section tapered toward the distal end. The material of the reinforcing member 120 is not particularly limited and may be a super-elastic alloy such as stainless steel (SUS304) or Ni-Ti alloy. The reinforcing member 120 extends through the outer-shaft middle portion 115 and then through the guidewire port 113 to reach the outer-shaft distal end 111. The reinforcing member 120 has a pusher 122 that can be come into contact with the guidewire port 113.

The interior of the balloon 90 accommodates two markers 105 attached to the outer circumference of the inner shaft 60a. This configuration allows a handler such as a doctor to keep track of the exact location of the balloon 90 during coronary angiography, leading to reliable dilation of a stenosis or stricture part or an obstructed part.

As shown in FIG. 10, the inner shaft 60a comprises, in order from inside in the radial direction, an inner layer 10e and the reinforcing layer (coil body) 30 that is disposed within the inner layer 10e and made of the wound wires 20 with each adjacent pair of the wires 20 having a gap 25b therebetween. The balloon 90 is bonded to the outer circumference of the inner shaft 60a and covers the reinforcing layer (coil body) 30.

In the portion where the inner shaft 60a is bonded to the balloon 90, in the gap 25b of the reinforcing layer (coil body) 30, disposed is a bonding portion 50f where an inner layer protruding portion 12f of the inner layer 10e sticking into the balloon 90 is engaged with a balloon protruding portion 92 of the balloon 90 sticking into the inner layer 10e.

The inner layer protruding portion 12f is composed of a proximal end direction extending portion 13f extending in the proximal end direction, an inwardly-extending portion 14f extending inwardly, and a distal end direction extending portion 15f extending in the distal end direction. The balloon protruding portion 92 is composed of an inwardly-protruding portion 93 extending inwardly, a distal end direction extending portion 94 extending in the distal end direction, an outwardly-extending portion 95 extending outwardly, and a proximal end direction extending portion 96 extending in the proximal end direction.

In the balloon catheter 6, in the gap 25b of the reinforcing layer (coil body) 30, disposed is the bonding portion 50f where the inner layer protruding portion 12f of the inner layer 10e sticking into the balloon 90 is engaged with the balloon protruding portion 92 of the balloon 90 sticking into the inner layer 10e. This configuration enlarges the area across which the balloon 90 and the inner layer 10e are bonded to each other within the gap 25b of the reinforcing layer (coil body) 30, leading to a strengthened bond between the balloon 90 and the inner layer 10e. The balloon 90 is anchored in the reinforcing layer (coil body) 30 to produce an anchoring effect, which can reduce the probability of the balloon 90 to come off the inner layer 10e when the balloon catheter 6 bends to cause the balloon 90 to be pulled in the axial direction (in the distal end direction and in the proximal end direction).

The engagement between the inner layer protruding portion 12f and the balloon protruding portion 92 of the bonding portion 50f can reduce the probability of the balloon 90 to come off the inner layer 10e when the balloon 90 is expanded radially (outwardly). In addition, the engagement between the inner layer protruding portion 12f and the balloon protruding portion 92 can allow a reduction in the thickness of the balloon 90 while maintaining the strength of the bond between the balloon 90 and the inner layer 10e, leading to an enhanced insertion of the balloon catheter 6 into a blood vessel, a bile duct, a pancreatic duct, or the like.

The inner shaft 60a of the balloon catheter 6 shown in FIG. 9 and FIG. 10 may be a combination of the inner layer 10, 10a, 10b, 10c, 10d of the catheter 1 to 5 according to the first embodiment to the fifth embodiment and the reinforcing layer (coil body) 30 or the reinforcing layer (braid) 35 disposed in the inner layer 10, 10c, 10d or on the outer circumference of the inner layer 10a, 10b.

The first bonding portion 50, 50a, 50b, 50c, 50d of the catheter 1 to 5 may have any shape provided that the first inner layer protruding portion 12, 12a, 12b, 12c, 12d and the first outer layer protruding portion 42, 42a, 42b, 42c are engaged with each other therein. The second bonding portion 50e of the catheter 5 may also have any shape provided that the second inner layer protruding portion 12e and the second outer layer protruding portion 82 are engaged with each other therein. In addition, the bonding portion 50f of the balloon catheter 6 may also have any shape provided that the inner layer protruding portion 12f and the balloon protruding portion 92 are engaged with each other therein.

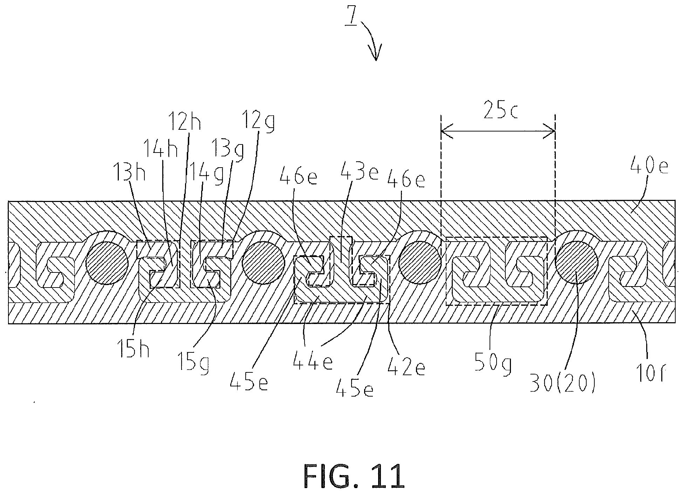

In a catheter 7 according to a seventh embodiment, which is illustrated in FIG. 11, in a gap 25c of the reinforcing layer (coil body) 30, disposed is a first bonding portion 50g where first inner layer protruding portions 12g, 12h of an inner layer 10f sticking into a first outer layer 40e is engaged with a first outer layer protruding portion 42e of the first outer layer 40e sticking into the inner layer 10f.

The first inner layer protruding portion 12g is composed of a distal end direction extending portion 13g extending in the distal end direction, an inwardly-extending portion 14g extending inwardly, and a proximal end direction extending portion 15g extending in the proximal end direction. The first inner layer protruding portion 12h is composed of a proximal end direction extending portion 13h extending in the proximal end direction, an inwardly-extending portion 14h extending inwardly, and a distal end direction extending portion 15h extending in the distal end direction. The first outer layer protruding portion 42e is composed of an inwardly-protruding portion 43e extending inwardly, an extending portion 44e extending in the distal end direction and in the proximal end direction, two outwardly-extending portions 45e extending outwardly, and two extending portions 46e extending respectively in the distal end direction or in the proximal end direction.

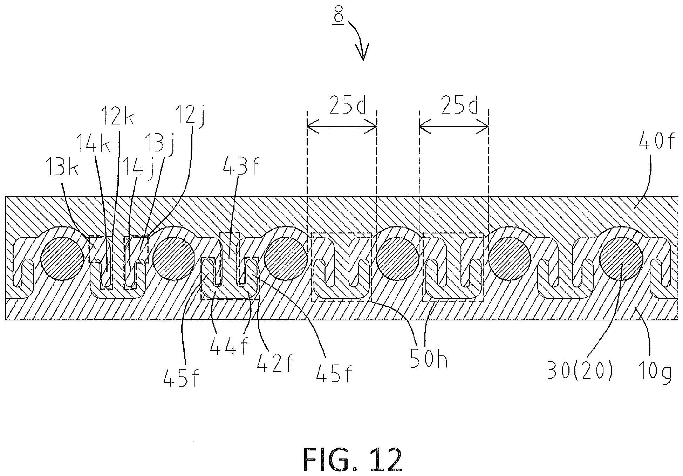

In a catheter 8 according to an eighth embodiment, which is illustrated in FIG. 12, in a gap 25d in the reinforcing layer (coil body) 30, disposed is a first bonding portion 50h where first inner layer protruding portions 12j, 12k of an inner layer 10g sticking into a first outer layer 40f is engaged with a first outer layer protruding portion 42f of the first outer layer 40f sticking into the inner layer 10g.

The first inner layer protruding portion 12j is composed of a distal end direction extending portion 13j extending in the distal end direction and an inwardly-extending portion 14j extending inwardly. The first inner layer protruding portion 12k is composed of a proximal end direction extending portion 13k extending in the proximal end direction and an inwardly-extending portion 14k extending inwardly. The first outer layer protruding portion 42f is composed of an inwardly-protruding portion 43f extending inwardly, an extending portion 44f extending in the distal end direction and in the proximal end direction, and two outwardly-extending portions 45f extending outwardly.

The reinforcing layer described above is the coil body 30 or the braid 35, but it is not limitative. For example, the reinforcing layer of any of the catheters 1 to 5, 7, and 8 and the balloon catheter 6 may be a hypotube (metal tube) having a helical slit serving as the gap.

### Reference Signs List

1 to 5, 7, 8: Catheter
6: Balloon catheter
10 to 10g: Inner layer
12 to 12d, 12f to 12h, 12j, 12k: First inner layer protruding portion
12e: Second inner layer protruding portion
20, 20a, 20b: Wire
25 to 25d: Gap
30, 35: Reinforcing layer
40 to 40f: First outer layer
42 to 42f: First outer layer protruding portion
50 to 50d, 50g, 50h: First bonding portion
50e: Second bonding portion
50f: Bonding portion
60: Catheter shaft
60a: Inner shaft
70, 100: Tip
75, 130: Connector
80: Second outer layer
82: Second outer layer protruding portion
90: Balloon
92: Balloon protruding portion
105: Marker
110: Outer shaft
120: Reinforcing member

## Claims

1. A catheter (1, 2, 3, 4, 5, 7, 8) comprising:
a tubular inner layer (10, 10a, 10b, 10c, 10d, 10f, 10g) a reinforcing layer (30, 35) disposed within the inner layer (10, 10a, 10b, 10c, 10d, 10f, 10g) or on an outer circumference of the inner layer (10, 10a, 10b, 10c, 10d, 10f, 10g), the reinforcing layer (30, 35) being made of wound wires (20, 20a, 20b), each adjacent pair of the wires (20, 20a, 20b) having a gap (25, 25a, 25c, 25d) therebetween; and
a first outer layer (40, 40a, 40b, 40c, 40d, 40e, 40f) covering the reinforcing layer (30, 35), **characterised in that**,
in the gap (25, 25a, 25c, 25d) of the reinforcing layer (30, 35), disposed is a first bonding portion (50, 50a, 50b, 50c, 50d, 50g, 50h) where a first inner layer protruding portion (12, 12a, 12b, 12c, 12d, 12g, 12h, 12j, 12k) of the inner layer (10, 10a, 10b, 10c, 10d, 10f, 10g) sticking into the first outer layer (40, 40a, 40b, 40c, 40d, 40e, 40f) is engaged with a first outer layer protruding portion (42, 42a, 42b, 42c, 42d, 42e, 42f) of the first outer layer (40, 40a, 40b, 40c, 40d, 40e, 40f) sticking into the inner layer (10, 10a, 10b, 10c, 10d, 10f, 10g).

2. The catheter (5) according to claim 1, further comprising:
a second outer layer (80) covering an outer circumference of the reinforcing layer (30), the second outer layer (80) being arranged on a distal end side of the first outer layer (40d), the second outer layer (80) being made of a resin that is more flexible than a resin of which the first outer layer (40d) is made, wherein:
in the gap (25) of the reinforcing layer (30), disposed is a second bonding portion (50e) where a second inner layer protruding portion (12e) of the inner layer (10d) sticking into the second outer layer (80) is engaged with a second outer layer protruding portion (82) of the second outer layer (80) sticking into the inner layer (10d),
a degree of the engagement of the second bonding portion (50e) is higher than a degree of the engagement of the first bonding portion (50d).

3. A balloon catheter (6) comprising:
a tubular inner layer (10e);
a reinforcing layer (30) disposed within the inner layer (10e) or on an outer circumference of the inner layer (10e), the reinforcing layer (30) being made of wound wires (20), each adjacent pair of the wires having a gap (25b) therebetween; and
a balloon (90) covering the reinforcing layer (30),
**characterised in that**,
in the gap (25b) of the reinforcing layer (30), disposed is a bonding portion (50f) where an inner layer protruding portion (12f) of the inner layer (10e) sticking into the balloon is engaged with a balloon protruding portion (92) of the balloon (90) sticking into the inner layer (10e).

## Patentansprüche

1. Katheter (1, 2, 3, 4, 5, 7, 8) mit:
einer röhrenförmigen Innenschicht (10, 10a, 10b, 10c, 10d, 10f, 10g),
einer innerhalb der Innenschicht (10, 10a, 10b, 10c, 10d, 10f, 10g) oder am Außenumfang der Innenschicht (10, 10a, 10b, 10c, 10d, 10f, 10g) angeordneten Verstärkungsschicht (30, 35), die aus einer Drahtwicklung (20, 20a, 20b) gebildet ist, wobei jedes nebeneinanderliegende Drahtpaar (20, 20a, 20b) einen dazwischenliegenden Abstand (25, 25a, 25c, 25d) hat; und
einer die Verstärkungsschicht (30, 35) ummantelnden ersten Außenschicht (40, 40a, 40b, 40c, 40d, 40e, 40f), **dadurch gekennzeichnet, dass**
im Abstand (25, 25a, 25c, 25d) der Verstärkungsschicht (30, 35) ein erster Verbindungsabschnitt (50, 50a, 50b, 50c, 50d, 50g, 50h) angeordnet ist, bei dem ein erster Innenschichtvorsprung (12, 12a, 12b, 12c, 12d, 12g, 12h, 12j, 12k) der Innenschicht (10, 10a, 10b, 10c, 10d, 10f, 10g), der in der ersten Außenschicht (40, 40a, 40b, 40c, 40d, 40e, 40f) steckt, in Eingriff ist mit einem ersten Außenschichtvorsprung (42, 42a, 42b, 42c, 42d, 42e, 42f) der ersten Au-βenschicht (40, 40a, 40b, 40c, 40d, 40e, 40f), der in der Innenschicht (10, 10a, 10b, 10c, 10d, 10f, 10g) steckt.

2. Katheter (5) nach Anspruch 1, des Weiteren mit:
einer einen Außenumfang der Verstärkungsschicht (30) ummantelnden zweiten Außenschicht (80), die auf der Seite eines distalen Endes der ersten Außenschicht (40d) angeordnet ist und aus einem Harz hergestellt ist, das biegsamer ist als ein Harz, aus dem die erste Außenschicht (40d) hergestellt ist,
wobei:
im Abstand (25) der Verstärkungsschicht (30) ein zweiter Verbindungsabschnitt (50e) angeordnet ist, bei dem ein zweiter Innenschichtvorsprung (12e) der Innenschicht (10d), der in der zweiten Außenschicht (80) steckt, in Eingriff ist mit einem zweiten Außenschichtvorsprung (82) der zweiten Außenschicht (80), der in der Innenschicht (10d) steckt, und
ein Eingriffsgrad des zweiten Verbindungsabschnitts (50e) höher ist als ein Eingriffsgrad des ersten Verbindungsabschnitts (50d).

3. Ballonkatheter (6) mit:
einer röhrenförmigen Innenschicht (10e);
einer innerhalb der Innenschicht (10e) oder am Außenumfang der Innenschicht (10e) angeordneten Verstärkungsschicht (30), die aus einer Drahtwicklung (20) gebildet ist, wobei jedes nebeneinanderliegende Drahtpaar einen dazwischenliegenden Abstand (25b) hat; und
einem die Verstärkungsschicht (30) ummantelnden Ballon (90), **dadurch gekennzeichnet, dass**
im Abstand (25b) der Verstärkungsschicht (30) ein Verbindungsabschnitt (50f) angeordnet ist, bei dem ein Innenschichtvorsprung (12f) der Innenschicht (10e), der in dem Ballon steckt, in Eingriff ist mit einem Ballonvorsprung (92) des Ballons (90), der in der Innenschicht (10e) steckt.

## Revendications

1. Cathéter (1, 2, 3, 4, 5, 7, 8), comprenant :
une couche interne tubulaire (10, 10a, 10b, 10c, 10d, 10f, 10g), une couche de renforcement (30, 35) disposée à l'intérieur de la couche interne (10, 10a, 10b, 10c, 10d, 10f, 10g) ou sur une circonférence externe de la couche interne (10, 10a, 10b, 10c, 10d, 10f, 10g), la couche de renforcement (30, 35) étant composée de fils enroulés (20, 20a, 20b), chaque paire adjacente des fils (20, 20a, 20b) présentant un espace (25, 25a, 25c, 25d) entre ceux-ci ; et
une première couche externe (40, 40a, 40b, 40c, 40d, 40e, 40f) recouvrant la couche de renforcement (30, 35), **caractérisé en ce que**
dans l'espace (25, 25a, 25c, 25d) de la couche de renforcement (30, 35), est disposée une première partie de liaison (50, 50a, 50b, 50c, 50d, 50g, 50h) dans laquelle une première partie saillante de couche interne (12, 12a, 12b, 12c, 12d, 12g, 12h, 12j, 12k) de la couche interne (10, 10a, 10b, 10c, 10d, 10f, 10g) s'enfonçant dans la première couche externe (40, 40a, 40b, 40c, 40d, 40e, 40f) est mise en prise avec une partie saillante de première couche externe (42, 42a, 42b, 42c, 42d, 42e, 42f) de la première couche externe (40, 40a, 40b, 40c, 40d, 40e, 40f) s'enfonçant dans la couche interne (10, 10a, 10b, 10c, 10d, 10f, 10g).

2. Cathéter (5) selon la revendication 1, comprenant en outre :
une seconde couche externe (80) recouvrant une circonférence externe de la couche de renforcement (30), la seconde couche externe (80) étant agencée côté extrémité distale de la première couche externe (40d), la seconde couche externe (80) étant composée d'une résine qui est plus flexible qu'une résine dont est composée la première couche externe (40d),
dans lequel :
dans l'espace (25) de la couche de renforcement (30), est disposée une seconde partie de liaison (50e) dans laquelle une partie saillante de seconde couche interne (12e) de la couche interne (10d) s'enfonçant dans la seconde couche externe (80) est mise en prise avec une partie saillante de seconde couche externe (82) de la seconde couche externe (80) mordant dans la couche interne (10d),
un degré de la mise en prise de la seconde partie de liaison (50e) est supérieur à un degré de la mise en prise de la première partie de liaison (50d).

3. Cathéter à ballonnet (6), comprenant :
une couche interne tubulaire (10e) ;
une couche de renforcement (30) disposée à l'intérieur de la couche interne (10e) ou sur une circonférence externe de la couche interne (10e), la couche de renforcement (30) étant composée de fils enroulés (20), chaque paire adjacente des fils présentant un espace (25b) entre ceux-ci ; et
un ballonnet (90) recouvrant la couche de renforcement (30), **caractérisé en ce que**,
dans l'espace (25b) de la couche de renforcement (30), est disposée une partie de liaison (50f) dans laquelle une partie saillante de couche interne (12f) de la couche interne (10e) s'enfonçant dans le ballonnet est mise en prise avec une partie saillante de ballonnet (92) du ballonnet (90) s'enfonçant dans la couche interne (10e).
